**Europäisches Patentamt**

**European Patent Office**

**Office européen des brevets**

(19)

(11) Veröffentlichungsnummer : **0 333 773 B1**

(12) **EUROPÄISCHE PATENTSCHRIFT**

(45) Veröffentlichungstag der Patentschrift :
**15.07.92 Patentblatt 92/29**

(51) Int. Cl.$^5$: **A41D 27/13,** A61L 15/16,
A61L 9/12

(21) Anmeldenummer : **88900947.8**

(22) Anmeldetag : **24.11.87**

(86) Internationale Anmeldenummer :
**PCT/EP87/00724**

(87) Internationale Veröffentlichungsnummer :
**WO 88/03765 02.06.88 Gazette 88/12**

(54) **SCHWEISSEINLAGE.**

(30) Priorität : **26.11.86 DE 3640374**

(43) Veröffentlichungstag der Anmeldung :
**27.09.89 Patentblatt 89/39**

(45) Bekanntmachung des Hinweises auf die
Patenterteilung :
**15.07.92 Patentblatt 92/29**

(84) Benannte Vertragsstaaten :
**AT BE CH DE FR GB IT LI LU NL SE**

(56) Entgegenhaltungen :
**EP-A- 0 201 134**
**DE-A- 2 004 872**

(56) Entgegenhaltungen :
**DE-A- 2 914 386**
**DE-A- 3 339 474**
**US-A- 3 490 454**
**US-A- 3 623 659**

(73) Patentinhaber : **Deotexis AG**
**Poststrasse 9**
**CH-6300 Zug (CH)**

(72) Erfinder : **Tebbe, Gerold**
**An der Staig 9**
**W-7968 Salgau (DE)**

(74) Vertreter : **Ostertag, Reinhard et al**
**Patentanwälte Dr. Ulrich Ostertag Dr.**
**Reinhard Ostertag Eibenweg 10**
**W-7000 Stuttgart 70 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine auswechselbare Schweißeinlage für Wäsche- oder Kleidungsstücke mit einer hautfreundlichen Saugschicht, welche z.B. Papiervlies, Faservlies, Filz, Zellstoff, Cellulosevlies oder eine Gewebe aus Wolle oder Baumwolle aufweisen kann, und mit einer dünnen wasserundurchlässigen Außenschicht, die mit Mitteln zum lösbaren Anbringen an einem Wäsche- oder Bekleidungsstück versehen ist, insbesondere einer selbstklebende Beschichtung.

Derartige Schweißeinlagen können z.B. den Deutschen Offenlegungsschriften 31 04 047, 33 04 749, 26 17 545, 28 41 365, 33 39 474, 32 36 941 und 32 47 859 entnommen werden. Sie dienen allgemein dazu, entstandenen Schweiß aufzusaugen.

Es sind ferner auch chemische Wirkstoffe bekannt, die gebildeten Schweiß so zersetzen, daß er geruchsneutral wird. Diese Wirkstoffe werden bisher direkt auf die Haut aufgebracht, wobei sich jedoch durch dieses direkte Aufbringen Grenzen für den Einsatz aus der Hautverträglichkeit ergeben.

Ferner finden in großem Umfange Deo-Sprays Verwendung, die schlechte Gerüche überdecken, allerdings nur für begrenzte Zeit. Darüber hinaus können Deo-Sprays ebenso wenig wie Deodorant-Stifte Schweißflecken an den Kleidungsstücken verhindern. Deo-Sprays sind darüber hinaus wegen ihrer Treibgase zum Teil umweltschädlich.

Das mechanische Angehen des Problems über eine Saugschicht, die ein Schweißreservoir bildet und ein Durchdringen des Schweißes in das Wäsche- oder Kleidungsstück oder durch dieses hindurch verhindert, hat auch weitere Nachteile: Die Saugschicht muß verhältnismäßig dick sein und ist daher im Tragen nicht besonders angenehm, und die in der Saugschicht gespeicherte Feuchtigkeit führt oft zu Entzündungen.

Versieht man derartige Schweißeinlagen zusätzlich mit einem Deodorant. so verdunstet dies in kurzer Zeit. Danach tritt der Schweißgeruch, bedingt durch die Ansammlung des Schweißes sogar noch vermehrt hervor.

Durch die vorliegende Erfindung soll eine auswechselbare Schweißeinlage für Wäsche- oder Kleidungsstücke geschaffen werden, welche über lange Zeit hinweg die Schweiß- und Geruchsbildung bekämpft.

Diese Aufgabe ist erfindungsgemäß gelöst durch eine auswechselbare Schweißeinlage, die dadurch gekennzeichnet ist, daß die Saugschicht schmiegsam ist und mikroverkapselte Duftstoffe und/oder mikroverkapselte schweißzersetzende und/oder die Schweißbildung hemmende und/oder entzündungshemmende Wirkstoffe enthält.

Durch die Mikroverkapselung wird erreicht, daß die Duftstoffe und Wirkstoffe erst dann freigegeben werden, wenn die Schweißeinlage an einem Wäsche- oder Kleidungsstück angebracht worden ist und dieses getragen wird. Die Schweißeinlage kann also ohne Einsiegelung für lange Zeit aufbewahrt werden, ohne daß sie ihre Wirksamkeit verliebt. Auch wenn das mit ihr versehene Wäsche- oder Kleidungsstück längere Zeit nicht getragen wird, verbraucht sich die Schweißeinlage nicht.

Mikroverkapselte Duftstoffe und mikroverkapselte entzündungshemmende Wirkstoffe sind zwar an sich bekannt, sie wurden bisher aber im Gebiet der Medizin und der Werbung (Duftstoffe freisetzende Rubbel-Druckfarben) verwendet. Entsprechende mikroverkapselte Stoffe werden von den Firmen EURAND in Mailand und NCR Dayton in Ohio/USA in den Handel gebracht. DE-A-2004872 und DE-A-2914386 weisen mikroverkapselte Stoffe auf, die aber für Damenbinden oder das gleichen benitat werden. Dabei wird das Problem der Schweigsamkeit der Einlagen nicht berüht.

Dadurch, daß die erfindungsgemäße Einlage mikroverkapselte Duft- und/oder Wirkstoffe enthält, braucht die Saugschicht nicht so reichlich dimensioniert zu werden, daß sie auch unter ungünstigen Bedingungen rein durch Aufsaugen von Schweiß eine Geruchsbildung verhindert. Sie kann vielmehr einen Teil dieser Aufgabe an die Duft- und/oder Wirkstoffe abgeben und braucht daher nur geringe Dicke zu haben, sodaß sie schmiegsam ist und sich unterschiedlich gewölbten Flächen leicht anpassen kann. Sie trägt auch nur wenig auf und kann auch unter dünnen Stoffen ohne Beeinträchtigung des Aussehens des Wäsche- oder Kleidungsstückes getragen werden.

Erfindungsgemäße Schweißeinlagen lassen sich schnell und mühelos an Wäsche- und Kleidungsstücken anbringen und von diesen wieder abnehmen. Sie hinterlassen an den Wäsche- und Kleidungsstücken keine häßlichen Klebestellen und behalten über Tage hinweg ihren angenehmen Duft und/oder ihre schweißhemmende oder schweißzersetzende Wirkung. Sie lassen sich gleichermaßen als Krageneinlage, als Einlage in der Unterwäsche, in der Sportbekleidung oder im Miederbereich, aber auch an Oberbekleidung wie Jackets oder Damenkleidern oder in Strümpfen oder Schuhen einsetzen.

Vorteilhafte Weiterbildungen der Erfindung sind in Unteransprüchen angegeben.

Mit der Weiterbildung der Erfindung gemäß Anspruch 2 wird erreicht, daß die Duftstoffe und/oder Wirkstoffe erst dann in größerem Ausmaße von den Mikrokapseln freigegeben werden, wenn eine vorgegebene Temperatur erreicht wird bzw. eine vorgegebene Menge Schweiß an die Mikrokapseln gelangt. Die Duft- und Wirkstoffabgabe wird somit automatisch abgeschaltet, wenn das Wäsche- oder Kleidungsstück nicht getragen wird.

Mit der Weiterbildung der Erfindung gemäß Anspruch 3 wird erreicht, daß die Freigabe der Duft- oder Wirkstoffe auch bei langer Temperatur- und Schweißeinwirkung noch anhält, da eine Teilmenge der Mikrokapseln erst nach langer Temperatur- oder Schweißeinwirkung die Duft- bzw. Wirkstoffe freigibt.

Die Weiterbildung der Erfindung gemäß Anspruch 4 läßt sich von der Herstellung her besonders einfach realisieren, auch sprechen Mikrokapseln unterschiedlicher Wandstärke im Prinzip in gleicher Weise auf Temperatur bzw. Schweiß an, so daß sich die Freigabecharakteristik über die Wandstärke in gut vorhersehbarer Weise vorgeben läßt.

In Fällen, in denen man zur Erzielung langer Resistenz der Mikrokapseln eine zu große Wandstärke vorsehen müßte, kann man gemäß Anspruch 5 für diejenigen der Mikrokapseln, die der Temperatur- oder Schweißeinwirkung längere Zeit standhalten sollen, eine zusätzliche Beschichtung aus einem anderen Material als dem Wand-Grundmaterial vorsehen.

Mit der Weiterbildung der Erfindung gemäß Anspruch 6 wird erreicht, daß beim normalen Tragen des Wäsche- oder Kleidungsstückes ohne nennenswerte Schweißbildung eine Grundmenge an Duftstoffen bzw. Wirkstoffen freigesetzt wird, wie sie sich aus der rein thermischen Aktivierung der Mikrokapseln ergibt. Bei erhöhter Schweißabsonderung wird aber rasch eine zusätzliche Duftstoffmenge bzw. Wirkstoffmenge bereitgestellt.

Auch die Weiterbildung der Erfindung gemäß Anspruch 7 dient der gezielten Erhöhung der Duftstoff- und Wirkstofffreigabe bei erhöhter körperlicher Anstrengung.

Wählt man die Saugschicht, die den weitaus überwiegenden Teil der Dicke der Einlage ausmacht, gemäß Anspruch 8 sehr dünn und die Konzentration mikroverkapselter Duft- und/oder Wirkstoffe groß, so eignet sich die Einlage insbesondere zur Anbringung an den Kragen von Hemden, an Wäschestücken und für dünne Kleider. Derartige Einlagen fallen optisch nicht auf, sind aber eine Hilfe für das Wohlbefinden und dienen der Schonung der Wäsche- oder Kleidungsstücke. Die Einlagen passen sich auch leicht unterschiedlich geformten Flächen an, und da sie in der Praxis mit eine Selbstklebeschicht versehen sind, können sie bei heißem Wetter oder großen Anstrengungen leicht ggf. auch öfters an einem Tag ausgewechselt werden.

Die Weiterbildung der Erfindung gemäß Anspruch 9 ist im Hinblick auf eine vorgegebene Grundabgabe an Duftstoffen für solche Zeiten von Vorteil, in denen keine erhöhte Schweißabgabe erfolgt.

Mit der Weiterbildung der Erfindung gemäß Anspruch 10 wird erreicht, daß ein Teil der schweißzersetzenden und/oder entzündungshemmenden Wirkstoffe solange zurückgehalten wird, bis eine größere Schweißbildung auftritt.

Die Weiterbildung der Erfindung gemäß Anspruch 11 ist im Hinblick auf eine möglichst verlustfreie Abgabe der schweißzersetzenden und/oder entzündungshemmenden Wirkstoffe an die Haut von Vorteil.

Die Weiterbildungen der Erfindung gemäß den Ansprüchen 12 und 13 sind im Hinblick auf gute faltenlose Anpassung auch an stark gekrümmte Flächen von Vorteil.

Nachstehend wird die Erfindung anhand von Ausführungsbeispielen unter Bezugnahme auf die Zeichnung näher erläutert. In dieser zeigen:

Figur 1: eine Aufsicht auf eine Schweißeinlage;

Figur 2: einen Schnitt durch eine Schweißeinlage;

Figuren 3 und 4: Aufsichten auf abgewandelte Schweißeinlagen;

Figuren 5 bis 9: Anwendungsbeispiele für die Schweißeinlagen nach den Figuren 1, 3 und 4;

Figur 10: einen stark vergrößerten Schnitt durch eine abgewandelte Schweißeinlage; und

Figur 11: Schnitte durch Mikrokugeln, welche Duft- oder Wirkstoffe enthalten und gegen Temperatur- und/oder Schweißeinwirkung unterschiedliche Resistenz aufweisen.

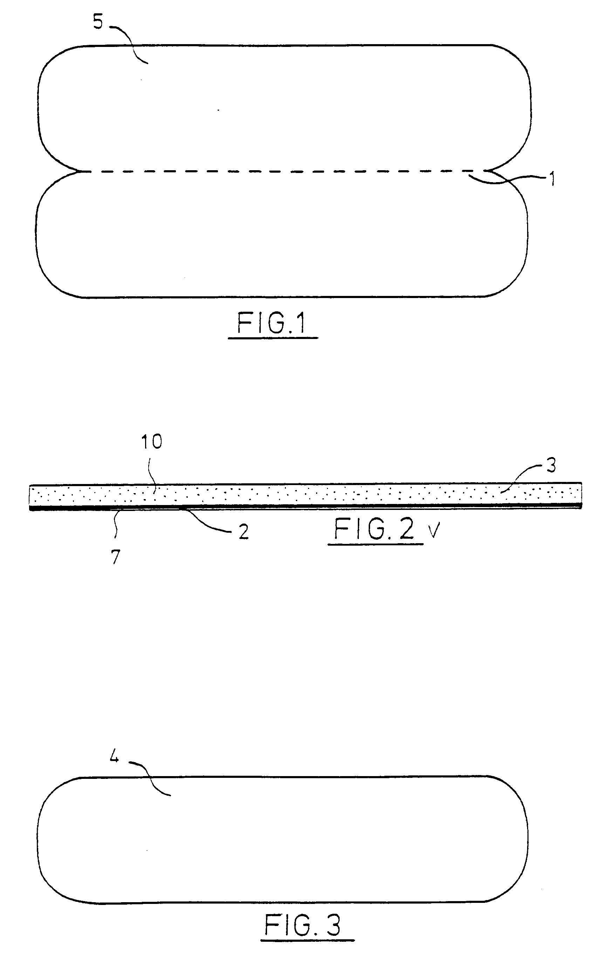

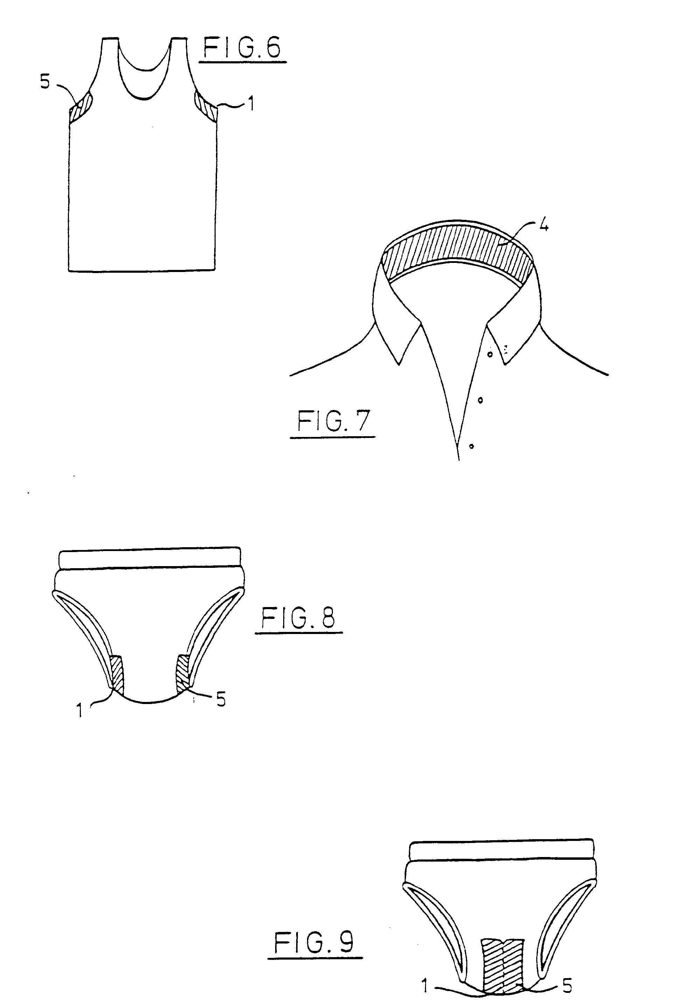

In Figur 1 ist eine Schweißeinlage insgesamt mit 5 bezeichnet. Sie ist aus einem Endlosmaterial unter Verwendung eines ihre Randkontur vorgebenden Stanzwerkzeuges ausgeschnitten und hat eine vorbereitete Falt- oder Knicklinie 1. Derartige Schweißeinlagen können z.B. zur Anbringung in Unterhemden, langen und kurzen Unterhosen und Slips dienen, wie in den Figuren 6, 8 und 9 angedeutet. Für diese Anwendungsfälle kann von der Faltlinie 1 Gebrauch gemacht werden (Figuren 6 und 8) oder nicht (Figur 9).

Die in Figur 1 gezeigte Schweißeinlage besteht aus einem Verbundmaterial, dessen Aufbau in Figur 2 näher gezeigt ist.

Auf einer dünnen Kunststoffolie 2 ist eine demgegenüber dicke, absolut aber immer noch dünne Saugschicht 3 fest angebracht, z.B. durch Verkleben oder Verschweißen. Die Saugschicht 3 besteht beispielsweise aus einem Papiervlies, Faservlies, Filz, Zellstoff, Cellulosevlies oder Gewebe aus Wolle oder Baumwolle, also saugfähigem und hautfreundlichem Material. Die Saugschicht 3 ist in Figur 2 als einlagige Schicht wiedergegeben, sie kann in der Praxis aber auch aus mehreren übereinander liegenden Lagen bestehen.

In die Saugschicht 3 sind in Figur 2 durch kleine Punkte angedeutete Mikrokapseln 10 eingebettet, und zwar beim Ausführungsbeispiel nach Figur 2 gleichförmig verteilt.

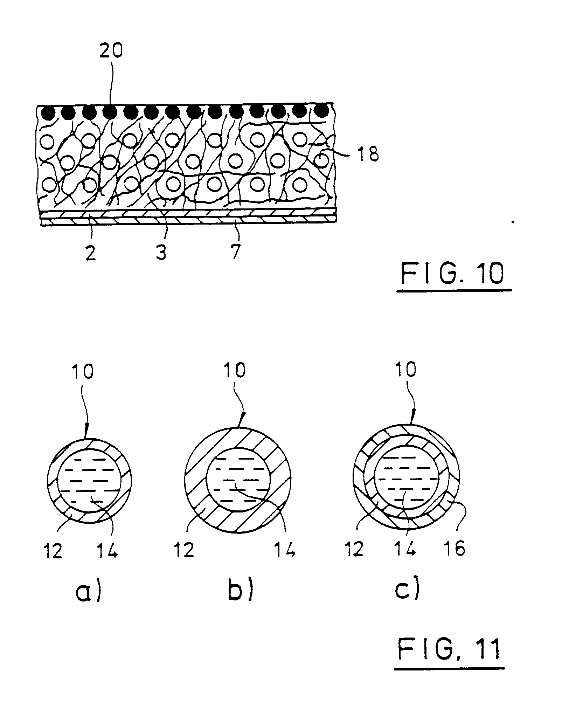

Wie in Figur 11 bei a) gezeigt, haben die in der Praxis einen Durchmesser von 5 bis 10 μ aufweisenden Mikrokapseln eine Wand 12, welche ein Flüssigkeitsvolumen 14 umschließt. Das Flüssigkeitsvolumen kann als Lösungsmittel für Duftstoffe und/oder schweißzersetzende und/oder die Schweißbildung hemmende Wirkstoffe dienen. Das Material, aus welchem die Wand 12 besteht, ist so ausgewählt, daß es sich bei Hautkontakt aufgrund der erhöhten Temperatur und/ oder durch Feuchtigkeitseinwirkung so verändert, daß die Duftstoffe und/ oder Wirkstoffe die Wand 12 durchsetzen können. Eine derartige Materialveränderung kann z.B. das völlige Auflösen der Wand 12 sein, wodurch das Flüssigkeitsvolumen 14 freigegeben wird. Die Wand 12 kann z.B. aus Gelatine bestehen.

Um zu verhindern, daß sich nicht alle der Mikrokapseln 10 gleichzeitig auflösen, kann man ein Gemisch von Mikrokapseln verwenden, wobei in diesem Gemisch das Material für die Wand 12 im Hinblick auf unterschiedliche Resistenz gegen Temperatur- und/oder Schweißeinwirkung gewählt ist. In diesem Falle kann dann die Wandstärke der verschiedenen Mikrokugel-Typen gleich groß sein.

Alternativ kann man gemäß Figur 11 b) einen Teil der Mikrokugeln mit vergrößerter Dicke der Wand 12 herstellen, wobei dann das Wandmaterial für alle Mikrokugeln gleich gewählt wird. Auch so erzielt man eine erhöhte Resistenz einer Teilmenge der Mikrokugeln gegen Temperatur- und/oder Schweißeinwirkung.

Figur 11 c) zeigt eine dritte Möglichkeit der Erhöhung der Resistenz für eine Teilmenge der Mikrokugeln durch Aufbringen einer Außenbeschichtung 16 aus einem Material, welches sich von demjenigen der Wand 12 unterscheidet.

Die in Figur 2 dargestellte Gleichverteilung der Mikrokugeln in der Saugschicht 3 ist für Duftstoffe einschließende Mikrokugeln vorteilhaft, ebenso für schweißzersetzende Wirkstoffe enthaltende Mikrokugeln. Für solche Mikrokugeln, die schon der Schweißentstehung entgegenwirkende Stoffe enthalten, ist es dagegen vorteilhaft, wenn sie möglichst nahe bei der freien Oberfläche der Einlage angeordnet werden, wie in Figur 10 schematisch wiedergegeben. Dort stellen Kreise solche Mikrokugeln 18 dar, die Duftstoffe und/ oder schweißzersetzende Wirkstoffe enthalten. Die Mikrokugeln 18 sind gleichförmig über die Saugschicht 3 verteilt. Schwarz ausgefüllte Kreisflächen stellen dagegen Mikrokugeln 20 dar, welche die Schweißbildung hemmende Wirkstoffe enthalten. Die Mikrokugeln 20 sind in der unmittelbaren Nachbarschaft der hautseitigen Oberfläche der Saugschicht 3 angeordnet. Die von ihnen abgegebenen Wirkstoffe gelangen somit gut zur Oberfläche der Schweißeinlage 3 und damit zur Haut.

Das Einbringen der Mikrokugeln 10 in die Saugschicht 3 kann auf trockenem Wege oder feuchtem Wege erfolgen. Im letztgenannten Falle kann man den Mikrokugeln noch ein Bindemittel zusetzen, welches ein gutes Haften der Mikrokugeln an den Fasern der Saugschicht 3 gewährleistet. Im erstgenannten Falle kann man die freie Oberfläche der Saugschicht 3 durch einen dünnen Strich verschließen, der z.B. dann gleich die nur an der Oberfläche vorzusehenden Mikrokugeln 20 enthalten kann.

Wie aus Figur 2 ersichtlich, ist auf die Unterseite der wasserundurchlässigen Kunststoffolie 2 eine Klebstoffschicht 7 aufgebracht, über welche die Schweißeinlage an einem Gewebe angebracht werden kann. Die Klebstoffschicht 7 braucht nicht durchgehend zu sein; sie kann auch eine Vielzahl von Klebstoffpunkten umfassen.

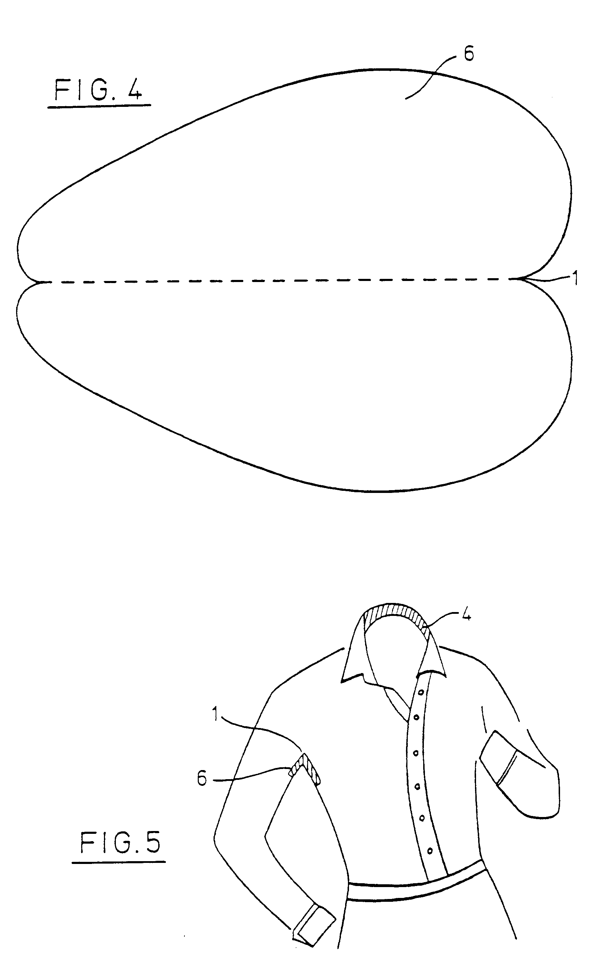

Figur 3 zeigt eine Schweißeinlage 4, wie sie in Verbindung mit Hemdenkragen verwendet werden kann. Auch diese Schweißeinlage ist mit einem geeigneten Stanzwerkzeug wieder aus einem Verbundmaterial ausgestanzt, wie es obenstehend unter Bezugnahme auf Figur 2 erläutert wurde. Einsatzmöglichkeiten dieser Schweißeinlage sind in den Figuren 5 und 7 gezeigt.

Figur 4 zeigt eine Schweißeinlage 6 für den Ärmelansatz eines Hemdes, die wieder mit einer die Anbringung erleichternden Knick- und Faltlinie 1 versehen ist. Ein Einsatzbeispiel der Schweißeinlage 6 ist in Figur 5 wiedergegeben.

Aus der obigen Beschreibung von Schweißeinlagen ist ersichtlich, daß diese sehr leicht an den unterschiedlichsten Wäsche- und Kleidungsstücken angebracht werden können, da die Saugschicht 3 insgesamt dünn und schmiegsam ist, ebenso die dünne Kunststoffolie 2, sodaß die gesamte Einlage leicht unterschiedlich geformt werden kann. Soll diese Formung absolut faltenfrei erfolgen, kann man die Saugschicht auch aus elastischem Material herstellen, wobei dann die Klebekraft der Klebstoffschicht 7 so hoch gewählt wird, daß die zur Formanpassung notwendige elastische Verformung auf Dauer aufgenommen werden kann. Umgekehrt kann man die Saugschicht 3 auch so aufbauen, daß sich die in ihr befindlichen Fasern bei Verformung der Saugschicht gegeneinander verschieben können. Auch auf diese Weise wird eine faltenfreie Anpassung an unterschiedlich gewölbte Flächen möglich.

Da nahezu jeder Duftstoff mikroverkapselt werden kann, kann man die oben beschriebenen Schweißeinlagen mit unterschiedlichen Duftnoten für die Benutzer bereitstellen, welche mit den Duftnoten von Parfums und Rasierwässern übereinstimmen.

Da, wie dargelegt, die Duftstoffabgabe und Wirkstoffabgabe erst mit dem Tragen eines mit der Schweißeinlage versehenen Wäsche- oder Kleidungsstückes einsetzt, kann man die Schweißeinlagen in größeren Pak-

kungen von 10 bis 100 Stück anbieten, wobei diese Packungen auch offen über lange Zeiträume gelagert werden können, ohne daß die Wirksamkeit der Schweißeinlagen leidet.

**Patentansprüche**

1. Auswechselbare Schweißeinlage für Wäsche- und Kleidungsstücke, mit einer hautfreundlichen Saugschicht, welche z.B. Papiervlies, Faservlies, Filz, Zellstoff, Cellulosevlies oder ein Gewebe aus Wolle oder Baumwolle aufweisen kann, und mit einer dünnen wasserundurchlässigen Außenschicht, die mit Mitteln zum lösbaren Anbringen an einem Wäsche- oder Kleidungsstück, insbesondere einer Selbstklebeschicht, versehen ist, dadurch gekennzeichnet daß die Saugschicht (3) schmiegsam ist und mikroverkapselte Duftstoffe (10) und/oder mikroverkapselte schweißzersetzende und/oder die Schweißbildung hemmende und/oder entzündungshemmende Wirkstoffe enthält.

2. Schweißeinlage nach Anspruch 1, dadurch gekennzeichnet, daß die Mikrokapseln (10) die Duftstoffe und/oder Wirkstoffe unter dem Einfluß von Körperwärme und/oder Schweiß freisetzen.

3. Schweißeinlage nach Anspruch 1 oder 2, dadurch gekennzeichnet, daß die Mikrokapseln ein Gemisch aus Mikrokapseln unterschiedlicher Resistenz gegen Körperwärme und/ oder Schweiß darstellen.

4. Schweißeinlage nach Anspruch 3, dadurch gekennzeichnet, daß die Mikrokapseln (10) ein Gemisch aus Kapseln aus gleichem Material jedoch unterschiedlicher Stärke ihrer Wand (12) darstellen.

5. Schweißeinlage nach Anspruch 2 oder 3, dadurch gekennzeichnet, daß ein Teil der Mikrokapseln (10) mit einer zusätzlichen Außenbeschichtung (16) aus sich bei Temperatur- und/ oder Schweißeinwirkung auflösendem Material aufweisen, welches sich vom Material der Innenwand (12) unterscheidet.

6. Schweißeinlage nach Anspruch 5, dadurch gekennzeichnet, daß die zusätzliche Beschichtung (16) ein sich bei Schweißeinwirkung rasch auflösendes Material aufweist.

7. Schweißeinlage nach einem der Ansprüche 1 bis 6, dadurch gekennzeichnet, daß die Mikrokapseln (10) über ein Bindemittel mit der Saugschicht (3) verbunden sind, welche sich bei Temperatur- und/oder Schweißeinwirkung auflöst.

8. Schweißeinlage nach einem der Ansprüche 1 bis 7, dadurch gekennzeichnet, daß die Saugschicht (3) sehr dünn ist, ein hohes Saugvermögen hat und die mikroverkapselten Duft- und/oder Wirkstoffe (10) in hoher Konzentration enthält.

9. Schweißeinlage nach einem der Ansprüche 2 bis 8, dadurch gekennzeichnet, daß zumindest ein Teil der Duftstoffmenge in allein durch Wärmeeinwirkung zur Abgabe aktivierbaren Mikrokapseln (10) eingeschlossen ist.

10. Schweißeinlage nach einem der Ansprüche 2 bis 9, dadurch gekennzeichnet, daß zumindest ein Teil der Wirkstoffe in nur durch Schweißeinwirkung zur Abgabe aktivierbaren Mikrokapseln (10) eingeschlossen ist.

11. Schweißeinlage nach einem der Ansprüche 1 bis 10, dadurch gekennzeichnet, daß die Schweißbildung hemmende Wirkstoffe enthaltende Mikrokapseln (20) überwiegend in der Nähe der freien Oberfläche der Saugschicht (3) angeordnet sind.

12. Schweißeinlage nach einem der Ansprüche 1 bis 11, dadurch gekennzeichnet, daß die Saugschicht (3) elastisch verformbar ist.

13. Schweißeinlage nach einem der Ansprüche 1 bis 11, dadurch gekennzechnet, daß die Saugschicht (3) plastisch verformbar ist, insbesondere leicht gegeneinander verschiebbare Fasern enthält, etwa in Form eines Vlieses.

**Claims**

1. Exchangeable anti-perspiration pad for underwear and articles of clothing, with an absorbent layer which is kind to the skin, which may comprise for example paper fleece, fibre fleece, felt, cellulose, celluloe fleece or a fabric of wool or cotton, and with a thin outer layer which is impervious to water, which is provided with means for the detachable application to underwear or articles of clothing, in particular a self-adhesive layer, characterised in that the absorbent layer (3) is flexible and contains micro-encapsulated odoriferous substances (10) and/or micro-encapsulated perspiration-decomposing and/or perspiration-inhibiting and/or anti-inflammatory active substances.

2. Anti-perspiration pad according to Claim 1, characterised in that the microcapsules (10) liberate the odoriferous substances and/or active substances under the influence of body heat and/or perspiration.

3. Anti-perspiration pad according to Claim 1 or 2, characterised in that the microcapsules represent a mixture of microcapsules of different resistance to body heat and/or perspiration.

4. Anti-perspiration pad according to Claim 3, characterised in that the microcapsules (10) represent a fixture of capsules of the same material, but with a different thickness of their walls (12).

5. Anti-perspiration pad according to Claim 2 or 3, characterised in that a part of the microcapsules (10) is provided with an additional outer coating (16) of material dissolving under the action of temperature and/or perspiration, which differs from the arterial of the inner wall (12).

6. Anti-perspiration pad according to Claim 5, characterised in that the additional coating (16) comprises a material dissolving quickly under the action of perspiration.

7. Anti-perspiration pad according to one of Claims 1 to 6, characterised in that the microcapsules (10) are connected to the absorbent layer (3) by a binder, which dissolves under the action of temperature and/or perspiration.

8. Anti-perspiration pad according to one of Claims 1 to 7, characterised in that the absorbent layer (3) is very thin, has a high absorption capacity and contains the micro-encapsulated odoriferous and/or active substances (10) in a high concentration.

9. Anti-perspiration pad according to one of Claims 2 to 8, characterised in that at least part of the quantity of odoriferous substance is enclosed in microcapsules (10) able to be activated for delivery solely by the action of heat.

10. Anti-perspiration pad according to one of Claims 2 to 9, characterised in that at least part of the active substances is enclosed in microcapsules (10) able to be activated for delivery solely by the action of perspiration.

11. Anti-perspiration pad according to one of Claims 1 to 10, characterised in that the microcapsules (20) containing the active substances inhibiting perspiration are provided predominantly in the vicinity of the free surface of the absorbent layer (3).

12. Anti-perspiration pad according to one of Claims 1 to 11, characterised in that the absorbent layer (3) is elastically deformable.

13. Anti-perspiration pad according to one of Claims 1 to 11, characterised in that the absorbent layer (3) is plastically deformable, in particular contains fibres which are able to move easily with respect to each other, possibly in the form of a fleece.

## Revendications

1. Pièce anti-transpiration amovible pour pièces de lingerie et d'habillement, avec une couche absorbante tolérable pour la peau qui peut comporter par exemple une nappe de papier, une nappe de fibres, un feutre, de la cellulose, une nappe de cellulose ou un tissu de laine ou de coton et avec une couche extérieure mince et imperméable à l'eau, qui comporte des moyens pour le placement de manière amovible sur une pièce de lingerie ou d'habillement, et en particulier une couche auto-collante, caractérisée en ce que la couche absorbante (3) est flexible et contient des substances odorantes (10) enrobées dans des micro-capsules et/ou des substances actives enrobées dans des micro-capsules, qui décomposent la transpiration et/ou qui empêchent la formation de transpiration et/ou qui empêchent l'irritation.

2. Pièce anti-transpiration selon la revendication 1, caractérisée en ce que les micro-capsules (10) dégagent les substances odorantes et/ou les substances actives sous l'effet de la chaleur du corps et/ou de la transpiration.

3. Pièce anti-transpiration selon la revendication 1 ou 2, caractérisée en ce que les micro-capsules constituent un mélange de micro-capsules ayant des résistances différentes à la chaleur du corps et/ou à la transpiration.

4. Pièce anti-transpiration selon la revendication 3, caractérisée en ce que les micro-capsules (10) constituent un mélange de capsules formées du même matériau mais ayant des parois (12) d'épaisseur différente.

5. Pièce anti-transpiration selon la revendication 2 ou 3, caractérisée en ce qu'une partie des micro-capsules (10) présente un revêtement extérieur supplémentaire (16) constitué d'une matière qui se dissout sous l'effet de la température et/ou de la transpiration et qui diffère de la matière de la paroi intérieure (12).

6. Pièce anti-transpiration selon la revendication 5, caractérisée en ce que le revêtement supplémentaire (16) comporte un matériau se dissolvant rapidement sous l'effet de la transpiration.

7. Pièce anti-transpiration selon l'une des revendications 1 à 6, caractérisée en ce que les micro-capsules (10) sont assemblées à la couche absorbante (3) par un liant qui se dissout sous l'effet de la température et/ou de la transpiration.

8. Pièce anti-transpiration selon l'une des revendications 1 à 7, caractérisée en ce que la couche absorbante (3) est très mince, présente un pouvoir absorbant élevé et contient les substances odorantes et/ou les substances actives (10) enrobées dans des micro-capsules et avec une concentration élevée.

9. Pièce anti-transpiration selon l'une des revendications 2 à 8, caractérisée en ce qu'au moins une partie de la quantité de substance odorante est enrobée dans des micro-capsules (10) pouvant uniquement être activées en vue du dégagement sous l'effet de la chaleur.

10. Pièce anti-transpiration selon l'une des revendications 2 à 9, caractérisée en ce qu'au moins une partie de substance active est enrobée dans des micro-capsules (10) pouvant uniquement être activées en vue du dégagement sous l'effet de la transpiration.

11. Pièce anti-transpiration selon l'une des revendications 1 à 10, caractérisée en ce que les micro-capsules (20) contenant les substances actives empêchant la formation de transpiration sont principalement disposées à proximité de la surface libre de la couche absorbante (3).

12. Pièce anti-transpiration selon l'une des revendications 1 à 11, caractérisée en ce que la couche absorbante (3) peut être déformée élastiquement.

13. Pièce anti-transpiration selon l'une des revendications 1 à 11, caractérisée en ce que la couche absorbante (3) peut être déformée élastiquement et contient en particulier des fibres pouvant facilement se déplacer les unes par rapport aux autres, un peu à la manière d'une nappe de fibres.

5

1

## FIG.1

10

3

7

2

## FIG. 2 ᵥ

4

## FIG. 3

FIG. 4

FIG. 5

FIG.6

5

1

4

FIG.7

FIG.8

1

5

FIG.9

1

5

FIG. 10

a)          b)          c)

FIG. 11